# EUROPEAN PATENT APPLICATION

(11) **EP 0 540 997 A1**
(43) Date of publication of application: **12.05.1993**
(21) Application number: 92118396.8
(22) Date of filing: 28.10.1992
(51) Int. Cl.: C12Q 1/68

(54) **Methods and reagents for HLA class I DNA typing**

(30) Priority: 05.11.1991 US 788113
(71) Applicant: F. HOFFMANN-LA ROCHE AG, CH-4002 Basel (CH)
(72) Inventor: Bugawan, Teodorica, San Leandro, California 94579 (US); Erlich, Henry A., Oakland, California 94602 (US)
(74) Representative: Notegen, Eric-André

(57) **Abstract**

Primers for amplification of specific nucleic acid sequences of the second and third exon of HLA Class I genes and probes for identifying polymorphic sequences contained in the amplified DNA can be used in processes for typing homozygous or heterozygous samples from a variety of sources and for detecting allelic variants not distinguishable by serological methods. This HLA Class I DNA typing system can be used in a forward or reverse dot-blot format that is simple and rapid to perform, produces detectable signals in minutes, and can-be used for tissue typing, determining individual identity, and identifying disease susceptible individuals.

Sequence Listing

(2) INFORMATION FOR SEQ ID NO: 1:
(i) SEQUENCE CHARACTERISTICS:
(A) LENGTH: 26 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 1:
GGCTGCAGCA CTCCATGAGG TATTTC 26
(2) INFORMATION FOR SEQ ID NO: 2:
(i) SEQUENCE CHARACTERISTICS:
(A) LENGTH: 27 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 2:
CAGGATCCCC TCGCTCTGGT TGTAGTA 27
(2) INFORMATION FOR SEQ ID NO: 3:
(i) SEQUENCE CHARACTERISTICS:
(A) LENGTH: 27 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 3:
GGCTGCAGTA CTACAACCAG AGCGAGG 27
(2) INFORMATION FOR SEQ ID NO: 4:
(i) SEQUENCE CHARACTERISTICS:
(A) LENGTH: 28 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 4:
CAGGATCCCT CCTTCCCGTT CTCCAGGT 28

(2) INFORMATION FOR SEQ ID NO: 5:
(i) SEQUENCE CHARACTERISTICS:
(A) LENGTH: 19 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 5:
GGATGTGAAG AAATACCTC 19
(2) INFORMATION FOR SEQ ID NO: 6:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 6:
AGGTATTTCT ACACCTCC 18
(2) INFORMATION FOR SEQ ID NO: 7:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 7:
AGGTATTTCT CCACATCC 18
(2) INFORMATION FOR SEQ ID NO: 8:
(i) SEQUENCE CHARACTERISTICS:
(A) LENGTH: 19 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 8:
GGATGTGGTG AAATACCTC 19

(2) INFORMATION FOR SEQ ID NO: 9:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 9:
CGGGACACGG ATGTGTAG 18
(2) INFORMATION FOR SEQ ID NO: 10:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 10:
TTCTACACCT CCGTGTCC 18
(2) INFORMATION FOR SEQ ID NO: 11:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 11:
GGACATGGCG GTGTCGAA 18
(2) INFORMATION FOR SEQ ID NO: 12:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 12:
TTCTACACCG CCATGTCC 18

(2) INFORMATION FOR SEQ ID NO: 13:
(i) SEQUENCE CHARACTERISTICS:
(A) LENGTH: 17 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 13:
CGGGACACGG CGGTGTA 17
(2) INFORMATION FOR SEQ ID NO: 14:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 14:
TTTCCACACC TCCGTGTC 18
(2) INFORMATION FOR SEQ ID NO: 15:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 15:
TTCCACACCG CCATGTCC 18
(2) INFORMATION FOR SEQ ID NO: 16:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 16:
GTATTGGGAC CAGGAGAC 18

(2) INFORMATION FOR SEQ ID NO: 17:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 17:
GTATTGGGAC GGGGAGAC 18
(2) INFORMATION FOR SEQ ID NO: 18:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 18:
GTATTGGGAC GAGGAGAC 18
(2) INFORMATION FOR SEQ ID NO: 19:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 19:
GTATTGGTAC CGGAACAC 18
(2) INFORMATION FOR SEQ ID NO: 20:
(i) SEQUENCE CHARACTERISTICS:
(A) LENGTH: 19 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 20:
CGTGTCTGXA GGTCCCAAT 19

(2) INFORMATION FOR SEQ ID NO: 21:
(i) SEQUENCE CHARACTERISTICS:
(A) LENGTH: 17 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 21:
TGTCTGXAGG TCCCAAT 17
(2) INFORMATION FOR SEQ ID NO: 22:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 22:
ACACGGAATA TGAAGGCC 18
(2) INFORMATION FOR SEQ ID NO: 23:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 23:
ACACGGAAAG TGAAGGCC 18
(2) INFORMATION FOR SEQ ID NO: 24:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 24:
GCCTTCACAT TCCGTGTC 18

(2) INFORMATION FOR SEQ ID NO: 25:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 25:
ACTGACCGAC GCAACCTG 18
(2) INFORMATION FOR SEQ ID NO: 26:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 26:
AGGTCCACTC GGTGAGTC 18
(2) INFORMATION FOR SEQ ID NO: 27:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 27:
AGGTCCACTC GGTCAGTC 18
(2) INFORMATION FOR SEQ ID NO: 28:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 28:
ACTGACCGAG CGAACCTG 18

(2) INFORMATION FOR SEQ ID NO: 29:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 29:
ATCGCGCTCC GCTACTAC 18
(2) INFORMATION FOR SEQ ID NO: 30:
(i) SEQUENCE CHARACTERISTICS:
(A) LENGTH: 19 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 30:
TGTGTTGGTC TTGAAGATC 19
(2) INFORMATION FOR SEQ ID NO: 31:
(i) SEQUENCE CHARACTERISTICS:
(A) LENGTH: 19 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 31:
ATCTCCAAGA CCAACACAC 19
(2) INFORMATION FOR SEQ ID NO: 32:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 32:
GCCTTGGCCT TGCAGATC 18

(2) INFORMATION FOR SEQ ID NO: 33:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 33:
GCGGAGGCCT TCAATTTC 18
(2) INFORMATION FOR SEQ ID NO: 34:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 34:
TACAAGGCCC AGGCACAG 18
(2) INFORMATION FOR SEQ ID NO: 35:
(i) SEQUENCE CHARACTERISTICS:
(A) LENGTH: 19 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 35:
AAGTACAAGC GCCAGGCAC 19
(2) INFORMATION FOR SEQ ID NO: 36:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 36:
GACCGGAACA CACAGATC 18

(2) INFORMATION FOR SEQ ID NO: 37:
(i) SEQUENCE CHARACTERISTICS:
(A) LENGTH: 18 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 37:
GATCTGTGTC TCCCGGTC 18
(2) INFORMATION FOR SEQ ID NO: 38:
(i) SEQUENCE CHARACTERISTICS:
(A) LENGTH: 19 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 38:
CTTCACATTC CGTGTGTTC 19
(2) INFORMATION FOR SEQ ID NO: 39:
(i) SEQUENCE CHARACTERISTICS:
(A) LENGTH: 19 bases
(B) TYPE: nucleic acid
(C) STRANDEDNESS: single
(D) TOPOLOGY: linear
(ii) MOLECULE TYPE: Other Nucleic Acid
(xi) SEQUENCE DESCRIPTION: SEQ ID NO: 39:
TGTGTTGGTC TTGCAGATC 19

## Description

The present invention provides methods and reagents for DNA typing HLA Class I nucleic acids. More particularly, the present invention relates to a method for determining the HLA Class I DNA type of nucleic acid in a sample, which method comprises:
(a) amplifying any nucleic acids in the sample that contain a Class I HLA allele second or third exon sequence;
(b) hybridizing said nucleic acid amplified in step (a) to a panel of oligonucleotide probes under conditions such that said probes hybridize only to exactly complementary sequences and
(c) determining from the pattern of probe hybridization in step (b) the Class I HLA alleles from which the nucleic acid in said sample originates. In another aspect, the present invention relates to a kit for performing the above method.

The invention enables one to type homozygous or heterozygous samples from a variety of sources, including samples comprising RNA or cDNA templates, and to detect allelic variants not distinguishable by present serological, cellular or biochemical methods. The present typing system facilitates typing tissue for transplantation, determining individual identity, and identifying disease susceptible individuals. The invention therefore has applications in the field of medicine generally and medical research and diagnostics specifically, the field of forensic science and the field of molecular biology.

The major histocompatibility complex (MHC) includes a number of genes that encode glycoproteins that, together with the T cell receptor (TCR), are the key element of specificity in the T cell response to foreign antigens. There are two structurally distinct, but related, families of MHC molecules that present antigens to two subsets of T cells: Class 1 MHC molecules present antigens to T cells that express the CD8 cell surface glycoprotein, and Class II MHC molecules present antigens to T cells that express the CD4 cell surface glycoprotein. See Bjorkman and Parham, 1990, Ann. Rev. Biochem. 59:253-288.

The HLA Class II proteins HLA DR, HLA DQ and HLA DP are encoded by genes in the major histocompatibitity complex (MHC) region on the short arm of human chromosome 6. The Class II proteins are heterodimeric glycoproteins consisting of an approximately 34 kD alpha chain and an approximately 29 kD beta chain. The Class II proteins are expressed on the cell surface of macrophages, B-cells, activated T-cells and other cell types and are involved in binding and presenting antigen to helper T-lymphocytes. See the article entitled "Structure, function and genetics of the Human Class II molecules" by Giles and Capra, 1985, Adv. Immunol. 37:1. In addition, the Class II proteins influence specific immune responsiveness by determining the repertoire of expressed T-cell receptors in mature T-lymphocytes. For a general review of the HLA Class II genes and proteins, see the article entiiled "Structure, sequence and polymorphism in the HLA D region" by Trowsdale et al., 1985, Immunol. Rev. 85:5.

Significant effort has been made to develop DNA based typing methods for determining the HLA Class II phenotype of an individual. See U.S. Patent Specification No. 4,582,788 which describes a restriction fragment length polymorphism (RFLP) based test. However, these RFLP-based analyses require large amounts of high molecular weight DNA, are labor intensive, and the limited number of informative restriction enzymes in turn limits the results obtained.

The advent of the polymerase chain reaction (PCR) has facilitated the analysis and manipulation of complex genomic DNA. The PCR process enables one to amplify a specific sequence of nucleic acid starting from a very complex mixture of nucleic acids and is more fully described in U.S. Patent Specifications Nos. 4,683,195; 4,683,202; 4,889,818 and 4,965,188 and in European Patent Publications Nos. 237,362 and 258,017. The PCR process has also facilitated typing the Class II HLA DNA of an individual. Scientists have studied the polymorphic second exon of DRB loci in genomic DNA by designing oligonucleotide primers and using those primers to amplify the sequences of interest. See the article entitled "Sequence analysis of the HLA DRB and HLA DQB loci from three Pemphigus vulgaris patients" by Scharf et al., 1988, Hum. Immunol. 22:61.

When the PCR primers contain restriction enzyme recognition sequences, the amplified DNA can be cloned directly into sequencing vectors and the nucleotide sequence of the amplification product can be readily determined. See the article entitled "Direct cloning and sequence analysis of enzymatically amplified genomic sequences" by Scharf et al., 1986, Science 233:1076. The amplified DNA can also be studied by detection methods that employ sequence specific oligonucleotide (SSO) probes. See the article entitled "Analysis of enzymatically amplified beta-globin and HLA DQalpha DNA with allele-speciflc oligonucleotide probes" by Saiki et al., 1986, Nature 324:163.

A number of important inventions in the field of Class II HLA DNA typing have led to the commercial development of simple tests for determining the genotype of an individual at a particular HLA Class II DNA locus. These inventions include those described in the European Patent Publication No. 237,362 and in the PCT Publications Nos. 89/04875, 89/11547, 89/11548 and 90/03444.

Despite the advances made in the field of Class II HLA DNA typing, very little progress has been made in developing methods for determining the Class I HLA DNA type of individuals. One reason for this lack of progress is the complexity of the HLA Class I genes. These genes encode the Class I A, B, C, E, F and G proteins, and although the F and G genes are currently not believed to be polymorphic, the A, B and C genes include at least 65 different sequences. The E gene exists in the population in 4 different DNA sequence variations. The currently known differences are primarily in the second and third exons of these genes (see Zemmour and Parham, 1991, Immunogenetics 33:310-320, although sequence variation in the fourth exon of these Class I genes is also known. See also Malissen et al., 1982, Proc. Natl. Acad. Sci USA 79:893-897.

These differences and the lack of knowledge concerning previously unknown variations in the sequence of Class I HLA genes have prevented the development of an informative and efficient means for determining the HLA Class I DNA type of an individual. The present invention meets the need for an efficient, informative Class I DNA typing method by providing novel processes and reagents. These novel processes and reagents will in turn lead to the discovery of previously unknown Class I alleles, which can also be typed and identified by the present method.

The present invention provides amplification and detection methods, generic and allele or group specific amplification primers, sequence specific oligonucleotide (SSO) probes, including probes for identifying previously unknown alleles at the Class I loci, and kits for practicing the methods, that together provide a rapid, simple and precise system for typing the alleles of the Class I HLA genes, including those that cannot be distinguished by serological methods.

In one aspect, the present invention provides a method for determining the Class I HLA DNA type of nucleic acid in a sample, which method comprises (a) amplifying any nucleic acids in the sample that contain a nucleic acid with a sequence of the second exon or third exon of a Class I HLA gene; (b) hybridizing said nucleic acid amplified in step (a) to a panel of oligonucleotide probes under conditions such that said probes hybridize only to exactly complementary sequences and (c) determining from the pattern of probe hybridization in steps (b) the HLA Class I alleles from which the DNA in said sample originates. As discussed below, a wide variety of methods exist for amplifying nucleic acids and determining the pattern of probe hybridization to nucleic acids in a sample.

In another aspect, the methods of the invention provide for the identification of the nucleic acids in a sample, and the phenotype of an individual from which that sample originated, with respect to any one, or any combination of, the HLA Class I genes. By appropriate choice of amplification reagents, and by adherence to the present methods, one can amplify and detect any particular second or third exon of choice from any of the Class I HLA genes. The present methods can also be used to determine fourth exon sequence variation in Class I HLA genes.

In a preferred embodiment, the present methods provide for the identification of the Class I HLA A, B and C nucleic acids in a sample. In a simplified format, the present methods and reagents provide for the identification of most known Class I HLA alleles of the A, B and C genes by probe hybridization to amplified nucleic acids containing second and/or third exon sequences from the Class I HLA A, B and C genes.

The present typing system can be used to type cDNA synthesized from mRNA and to type and study the expression of HLA Class I genes in tissues, transgenic systems, disease states and cells lines. Cells that do not express the Class I antigens or show unusual seroreactivity, such as tumor cells, can be readily typed. Moreover, samples from unusual sources, e.g., ancient DNAs or forensic samples, can be typed, even when the DNA sample is degraded or when only very small quantities are available for analysis.

Because PCR can amplify a fragment of target DNA over a million-fold, and because the present system can employ PCR-generated nucleic acid, radioactively labeled probes are not necessary, and nonisotopic SSOs covalently coupled to horseradish peroxidase (HRP) provide sufficient sensitivity for detection. The presence of the specifically bound HRP-labeled probes of the invention can be detected in a simple dot-blot format by chromogenic dye or chemiluminescent substrates in a matter of minutes.

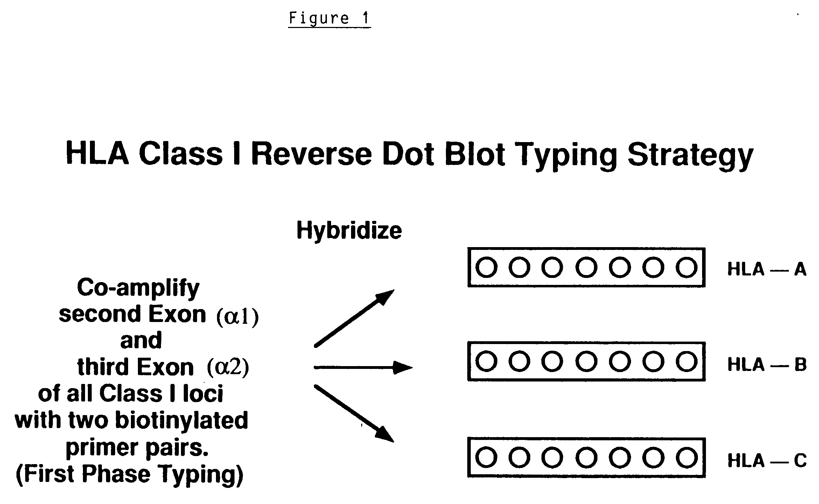

Figure 1 shows one embodiment of the invention in which two primer pairs and multiple membrane strips with immobilized probes identify the Class I HLA A, B and C alleles.

The present invention provides a Class I HLA DNA typing system and sequence specific oligonucleotide probes (SSOs) for analyzing Class I alleles. The invention can be used to type heterozygous samples from a variety of sources, including cDNA templates, and can be used to detect allelic variants not distinguishable by serological methods. This typing system can utilize a dot-blot format that is simple and rapid to perform, produces detectable signals in minutes and will prove valuable for tissue typing and determining individual identity and disease susceptibility.

In one embodiment, the invention provides a method for distinguishing between any one of the alleles of the Class I HLA genes present in a sample that have been sequenced at the DNA level, including 22 Class I HLA A alleles, 31 B alleles, 12 C alleles and 4 E alleles. Those of skill in the art recognize that over 50 different HLA B serologic types are known to exist, and the present invention provides a method for identifying HLA B alleles that have not been sequenced yet contribute to the number of different serological types. The total number of different HLA B alleles will probably be over 100 within a short time after the present invention becomes widely practiced, and new alleles are discovered. Similarly, previously unknown alleles of the other HLA Class I genes will be discovered by practice of the present invention. In a preferred embodiment, four PCR primers and several oligonucleotide probes provide for the identification of many different A and B second and third exon differences uniquely characteristic of the A and B alleles. The pattern of probe hybridization can be scanned and analyzed with computer assistance, facilitating the identification of the Class I alleles from which nucleic acid in a sample originates.

The diversity of the Class I HLA genes and the large number of alleles of these genes in the population make difficult the process of identifying the particular HLA Class I alleles from which a nucleic acid in a sample originates. The present invention allows one to make this determination with great specificity and so can be used to identify the particular individual from whom a sample was taken. This discrimination power in turn leads to the applications of the invention in the field of forensic science.

Because PCR (or other amplification processes such as ligase chain reaction or Q-beta replicase amplification) can be used to amplify very small amounts of DNA (or degraded DNA), the present invention can be used to type HLA DRB DNA from unusual sources, such as a buccal swab, a single hair, and even DNA from preserved ancient specimens. With the latter samples, analysis of the alleles from prehistoric sources, e.g., early hominids, is possible. For purposes of the present invention, "amplification" is defined by any process that increases the amount of target nucleic acid in a sample by means of nucleic acid replication or transcription.

Thus, amplification systems that can be used in the method of the present invention include those systems described in European Patent Publications Nos. 368,906; 310,229; 359,789; 358,737; 329,822; 386,228; 369,775; 373,960 and 408,295; and in PCT Patent Publications Nos. 90/06376; 90/03445; 90/02820; 90/02819 and 90/01068.

The present method can be used for Class I HLA DNA typing of cells that do not express the HLA Class I genes. The method is also suitable for DNA typing of cDNA synthesized from mRNA. The latter method facilitates the study of the expression of HLA genes in various cell lines or tissues and can be used to determine if there is an association between HLA gene expression and susceptibility to transformation, autoimmunity or other health conditions.

The research potential of the present invention should in no way obscure the immediate clinical applications. The genes and gene products of the MHC play a central role in the immunological state of an individual, and particular MHC gene products are associated with disease resistance and susceptibility. Because the present invention allows the determination of the MHC Class I genes in a sample, the invention also has applications in the field of medicine, particularly for medical diagnostic methods.

The discriminating power of this system will be valuable in typing potential transplantation donors, where very precise HLA matching appears to be critical in minimizing risk of rejection or graft versus host disease. In addition to the above benefits, the present invention also provides methods for identifying previously unknown HLA Class I alleles, and related primers, probes and methods for the identification of any Class I allele.

The present invention also provides kits for making practice of the present typing method more convenient. One type of kit includes both amplification and typing reagents. Another kit contains only one or more probes of the invention. In either kit, the probes can be labeled or unlabeled or attached to a solid support. The primers, if present in the kit, can also be labeled to facilitate detection, i.e., to bind a signal development reagent or for immobilization, or both. The kits can also contain reagents that facilitate detection of probe hybridization, i.e., the chromogenic substrate TMB and streptavidin-linked horseradish peroxidase. In brief, the reagents useful in practicing the present method can be packaged in any configuration that promotes utilization of the invention.

In a preferred embodiment, the present reagents comprise two or more different PCR primers selected from the group consisting of DB308 (SEQ IN NO: 1), DB309 (SEQ IN NO: 2), DB311 (SEQ IN NO: 3), and DB337 (SEQ IN NO: 4). These primers are shown in Table 1 below.

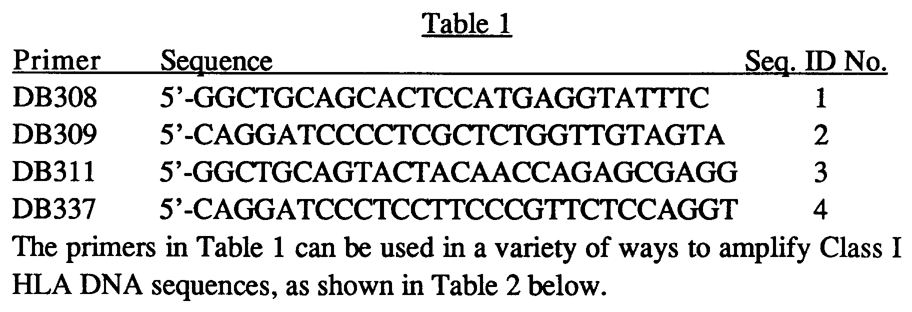

**Table 2**

| Primer | Hybridization Location | Amplification Product Size |
|---|---|---|
| | | With Primer |
| DB308 (SEQ IN NO: 1) | Left Side or Start of Exon 2 | With DB309 (SEQ IN NO: 2), about 270 base pairs (bp) |
| | | With DB337 (SEQ ID NO: 4), about 900 bp |
| DB309 (SEQ IN NO: 2) | Right Side or End of Exon 2 | With DB308 (SEQ IN NO: 1), about 270 bp |
| DB311 (SEQ IN NO: 3) | Left Side or End of Exon 2 | With DB337 (SEQ IN NO:4), about 500 bp |
| DB337 (SEQ IN NO: 4) | Right Side or End of Exon 3 | With DB308 (SEQ IN NO: 1), about 900 bp |
| | | With DB311 (SEQ IN NO: 3), about 500 bp |

By choosing the appropriate primer pair, one can use the primers of Table 2 to amplify any second and/or third exon sequence of any Class I HLA gene.

As noted above, the extensive allelic diversity of the HLA Class I genes is localized not only in the second exon, like that of the Class II beta genes, but also in the third exon. In general, the pattern of second and third exon sequence polymorphism, when present in the population at a particular locus, is a patchwork, with specific polymorphic segments found in a variety of different alleles. In principle, such shared epitopes among different alleles could reflect either common ancestry, gene conversion, or convergent evolution. For purposes of oligonucleotide typing, however, this patchwork pattern of polymorphism means that many alleles cannot be identified by hybridization to a single oligonucleotide but can be identified by a unique pattern of hybridization with a panel of probes.

In some instances, however, one merely wants to identify whether any allele of a particular Class I HLA gene is represented in the nucleic acids in a sample. The present methods can be used to amplify nucleic acids containing Class I HLA sequences, and the genes from which those nucleic acids originated can be identified using locus specific probes. These locus specific probes of the invention will hybridize to any allele of a particular Class I HLA gene but will not hybridize (under the appropriate conditions) to alleles of other Class I genes. Locus specific probes can also be prepared as mixtures of different probes or will be hybridized under conditions that do not demand complete sequence specific hybridization.

The sequence specific oligonucleotide probes of the invention, called "SSOs," when employed in the present methods under the appropriate hybridization and wash conditions, are extremely specific, capable of distinguishing single nucleotide polymorphisms. The SSOs of the invention, unlike the probes used in RFLP methods, can be used to determine not only if alleles are different but also where and how the alleles differ. The horseradish peroxidase-conjugated SSOs of the invention, called "HRP-SSOs," allow detection methods that employ chromogenic or chemiluminescent substrates that are easy to use and produce detectable signals rapidly (typically 1 to 10 minutes). The HRP-SSOs are stable for over two years without detectable loss of activity when stored at 4°C. See the article entitled "Nonisotopically labeled probes and primers" by Levenson and Chang, 1989, in PCR Protocols: A Guide to Methods and Applications (Innis, Gelfand, Sninsky and White ed., Academic Press, Inc. SanDiego). Radiolabelled probes can be employed but are not necessary for excellent sensitivity, an important benefit provided by the present invention.

In a preferred embodiment, the method of the present invention is practiced with a panel of probes for typing the HLA Class I A and B alleles. In the Table, the "region" identifies the polymorphic codons of the second exon of the Class I HLA A or B alleles to which the probe hybridizes. Region A includes codons 9 to 12 of exon 2 of both A and B alleles. Region B includes codons 62 and 63 of exon 2 of A alleles. Region C includes codons 65 to 67 of exon 2 of A alleles and codons 69 to 71 of exon 2 of B alleles. Region D includes codons 73 to 77 of exon 2 of A alleles. These probes, regions and alleles are shown in Table 3 below.

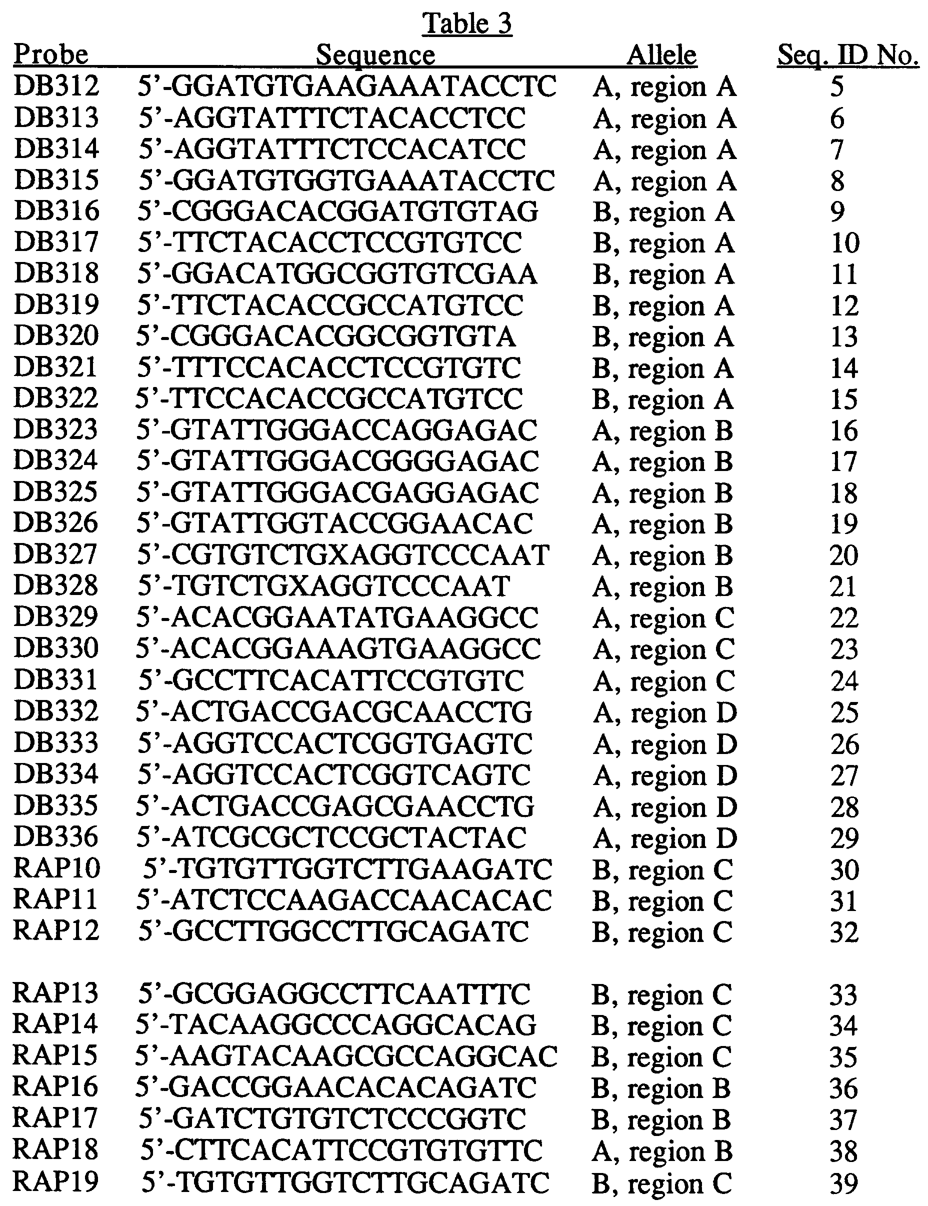

In the Table above, X represent an inosine nucleotide.

The dot-blot format for detection enables the rapid typing of a large number of samples and will be useful in determining the allele frequencies of Class I HLA genes. A recently developed alternative for PCR/SSO typing is the immobilized reverse dot blot format. See the article entitled "Genetic analysis of amplified DNA with immobilized sequence-specific oligonucleotide probes" by Saiki et al., 1989, Proc. Natl. Acad. Sci. USA 86:6230. In this procedure, the SSO probes are applied and fixed to the filter (rather than the amplified DNA applied and fixed to the filter), hence the term "reverse dot blot."

The reverse dot blot procedure allows a single sample to be analyzed in a single hybridization with a membrane containing an array of immobilized probes. The conventional dot blot format is useful when the number of samples exceeds the number of probes used (e.g., patient versus control or population genetics studies). The reverse dot blot format is valuable for clinical, diagnostic and forensic analyses, where there are fewer samples than probes. The reverse dot blot format is described in more detail below.

The following examples show illustrative preferred embodiments of the present invention. The examples show that the present invention provides, in a preferred embodiment, a nonisotopic PCR/SSO system for HLA Class I DNA typing that is simple, rapid and capable of precise DNA typing for a variety of samples from different sources.

### Example 1

### Amplification and Detection Methods

For typing samples, about 0.5 µg of human genomic DNA was amplified using reaction conditions as described in the article entitled "Primer-directed enzymatic amplification of DNA with a thermostable DNA polymerase," by Saiki et al., 1988, Science 239:487 and in Scharf et al., 1988, Hum. Immunol 22:61, and Scharf et al., 1989, Proc. Natl. Acad. Sci. USA 86:6215. The primers were present in the reaction mixture at 500 nM, and the reaction volume was 100 µg.

For sequencing the HLA alleles, 1 µg of purified human genomic DNA is amplified, and the amplified DNA is cloned into M13mp10 by the methods described in Scharf et al., 1988, Hum. Immunol. 22:61, and Scharf et al., 1986, Hum. Immunol. 233:1076. The inserts are then sequenced by the dideoxy chain-termination procedure described in Sanger et al., 1977, Proc. Natl. Acad. Sci. USA. 74:5463.

Samples were amplified for 30 to 35 cycles using the reaction conditions shown below. About 5 units (rather than 2.5 units) of Taq polymerase were added per 100 µl of reaction volume. RNA is amplified as described (see Kawasaki, 1989, "Amplification of RNA" in PCR Protocols: A Guide to Methods and Applications (Innis et al., eds., Academic Press, San Diego). All samples were overlaid with 100 µl of high grade mineral oil to prevent evaporation. After amplification, the oil overlay was extracted with 100 µl chloroform.

The thermal profile for each cycle comprised incubations at the following temperatures for the indicated times: 1 minute at 95°C (for denaturation of the DNA strands), 30 seconds at 60°C (for annealing of the primers), and 1 minute at 72°C (for extension of the primed templates). After the last cycle, the thermal cycler was programmed to incubate the samples at 72°C for 10 minutes to ensure that the final extension was complete. Care should be taken to avoid cross-contamination of samples; one must particularly guard against allowing the product of one PCR to contaminate an unamplified sample.

After amplification, a small portion of the amplified DNA is denatured and applied to a series of nylon filters; each filter is then hybridized to one of the labelled probes. Each SSO probe can be covalently conjugated to horseradish peroxidase (HRP) and provides a means of nonisotopic detection in the presence of a chromogenic or chemiluminescent substrate.

Thus, 5 µl of each amplified DNA sample are mixed with 100 µl of a mixture composed of 0.4 M NaOH and 25 mM EDTA, and the resulting mixture applied to BioDyne B nylon filters (Pall Corp., Glen Cove, NY) using a dot-blot manifold (Bio Rad, Richmond, CA). The filters, still in the dot-blot manifold, are rinsed with a mixture of 10 mM Tris-HCl and 0.1 mM EDTA, at pH 8.0, and dried on Whatman 3MM paper. The DNA is immobilized on the nylon filter by ultraviolet irradiation at a flux of 55 mJ/cm² with a Stratalinker™ (Stratagene, La Jolla, CA) UV light box.

Unless otherwise noted, all filters are hybridized in 2X SSPE (saline sodium phosphate EDTA), 5X Denhardt's solution, and 0.5% SDS with 2 pmoles of HRPSSO probe per 5 ml of hybridization solution for 15 min. at 42°C. Horseradish peroxidase conjugated oligonucleotides are prepared as described by Levenson and Chang, 1989, in PCR Protocols: A Guide to Methods and Applications (Innis et al., eds., Academic Press, Inc. San Diego) and Saiki et al., 1988, N. Eng. J. Med. 319:537. Filters for each probe are washed in SSPE.

After washing, filters to be developed with a chromogenic dye substrate are rinsed in PBS at room temperature for 30 minutes, then placed in 100 mM sodium citrate, pH 5.0, containing 0.1 mg/ml of 3,3',5,5'-tetramethylbenzidine (TMB) and 0.0015 percent hydrogen peroxide, and incubated with gentle agitation for 5 to 15 minutes at room temperature. Developed filters are rinsed in PBS and immediately photographed. Filters that are developed with the chemiluminescent detection system (ECL; Amersham, Arlington Heights, IL) are rinsed in PBS for 5 minutes and placed in the ECL solution for 1 minute with gentle agitation. Filters are then exposed to X-ray film at room temperature for 1 to 5 minutes.

### Example 2

### Reverse Dot Blot Format

In this embodiment of the invention, the Class I HLA probes are fixed to a membrane, and the amplified target DNA is hybridized to the membrane-bound probe. The set of typing probes is designed so that each probe will hybridize to a specific target sequence at the same temperature and salt concentration (and stay hybridized under the same wash conditions) as all other probes in the set. The PCR primers used in the amplification are biotinylated, as described in the book PCR Protocols, so that any amplified DNA that hybridizes to the membrane-bound probes can be easily detected. Those of skill in the art recognize that methods other than the method of the present example can be used to determine whether a probe has hybridized to a target nucleic acid.

In one embodiment, detection is carried out by reacting streptavidin (SA)conjugated horseradish peroxidase with any biotinylated, amplified DNA hybridized to the membrane-bound probe. The HRP thus becomes bound, through the SA-biotin interaction, to the amplified DNA and can be used to generate a signal by a variety of well know means, such as the generation of a colored compound, e.g., by the oxidation of tetramethylbenzidine.

Although the probes can be fixed to the membrane by any means, a preferred method involves "tailing" an oligonucleotide probe about 13 to 25 nucleotides in length with a much longer sequence of poly-dT. The resulting poly-dT "tail" can then be reacted with amine groups on the membrane to fix the probe covalently to the membrane. This reaction can be facilitated by UV irradiation.

Terminal deoxyribonucleotidyl transferase (TdT, Ratliff Biochemicals; for the reactions below assume a concentration of about 120 Units/µl, which is 100 pmol/µl) can be used to create a poly-dT tail on a probe, although one can also synthesize the tailed probe on a commercially available DNA synthesizer. When one uses a DNA synthesizer to make the tailed probe, however, one should place the tail on the 5' end of the probe, so that undesired premature chain termination occurs primarily in the tail region.

TdT reactions should be carried out in volume of about 100 µl containing 1X TdT salts, 200 pmol of oligonucleotide, 800 M dTT, and 60 units of TdT. 10X TdT salts is 1,000 mM K-cacodylate, 10 mM CoCl₂, 2 mM dithiothreitol, 250 mM Tris-Cl, pH 7.6, and is prepared as described by Roychoudhury and Wu, Meth. Enzymol. 65:43-62, incorporated herein by reference. A 10X stock solution of 8 mM dTTP can be prepared (neutralized to pH 7 with NaOH) for convenience.

The TdT reaction should be carried out at 37°C for two hours and then stopped by the addition of 100 µl of 10 mM EDTA, pH 8. The final concentration of tailed oligonucleotide is 1 µM (1 pmol/µl), and the length of the homopolymer tail is about 400 residues. Tail length can be changed by adjusting the molar ratio of dTTP to oligonucleotide. The tailed probes can be stored at -20°C until use.

Two types of nylon membrane are preferred for the reverse dot blot format: Biodyne™ nylon membrane, 0.45 micron pore size, manufactured by Pall; and Biotrans™ nylon membrane, 0.45 micron pore size, manufactured by ICN. The probes can be spotted onto the membrane very conveniently with the Bio-Dot™ dot blot apparatus manufactured by BioRad. Each probe is spotted onto a unique, discrete location onto the membrane. About 5 to 10 picomoles of each tailed probe is premixed with 60-100 µl of TE buffer before application to the dot blot apparatus. After dot blotting, the membrane is briefly placed on absorbent paper to draw off excess liquid.

The membrane is then placed inside a UV light box, such as the Stratalinker™ light box manufactured by Stratagene, and exposed to 50 to 60 millijoules of flux to fix the tailed probe to the nylon membrane. After a brief rinse (for about 15 minutes in hybridization solution) to remove unbound probe, the membrane is then ready for hybridization with biotinylated PCR product. One-half to one picomole (one-quarter to one-half of a typical, 100 µl PCR mixture) of PCR product is added to each probe panel for hybridization. About 50 µl of commercially available streptavidin-horseradish peroxidase conjugate can be added at this time for convenience, but better signals will result if a separate SA-HRP incubation and wash, at room temperature, is performed after the stringency wash.

Hybridization is typically carried out at 50 to 55°C for 30 minutes in a water bath and with hybridization buffer composed of 0.5% SDS and 3X to 5X SSPE, most commonly 4X. Stringency washing is carried out at 50 to 55°C for 15 minutes in a water bath and with wash solution composed of 0.1% SDS and 1X SSPE. A postwash of 1X PBS at room temperature for 30 minutes can enhance signal quality.

In a preferred embodiment, the sample is typed by amplification with all four primers DB308 (SEQ ID NO: 1), DB309 (SEQ ID NO: 2), DB337 (SEQ ID NO: 4), and DB311 (SEQ ID NO: 3), resulting in coamplification of both second and third exon Class I HLA nucleic acid sequences and then hybridized to four sets of probes, each set on a different membrane strip: an A second exon set, a B second exon set, an A third exon set, and a B third exon set of probes.

## Claims

1. A method for determining the HLA Class I DNA type of nucleic acid in a sample, which method comprises:
(a) amplifying any nucleic acids in the sample that contain a Class I HLA allele second and/or third exon sequence;
(b) hybridizing said nucleic acid amplified in step (a) to a panel of oligonucleotide probes under conditions such that said probes hybridize only to exactly complementary sequences and
(c) determining from the pattern of probe hybridization in step (b) the Class I HLA alleles from which the nucleic acid in said sample originates.

2. The method according to Claim 1, wherein said Class I HLA alleles are selected from the group consisting of the A, B and C alleles.

3. The method according to Claim 2, wherein said Class I HLA alleles are the A and B alleles.

4. The method according to any one of Claims 1-3, wherein the amplification of step (a) is carried out by a polymerase chain reaction with a pair of oligonucleotide primers.

5. The method according to Claim 4, wherein said second exon sequences are amplified.

6. The method according to Claim 5, wherein said primers are DB308 (SEQ ID NO: 1) and DB309 (SEQ ID NO: 2).

7. The method according to Claim 4, wherein said third exon sequences are amplified.

8. The method according to Claim 7, wherein said primers are DB337 (SEQ ID NO: 4) and DB311 (SEQ ID NO: 3).

9. The method according to Claim 4, wherein both said second and said third exon sequences are amplified.

10. The method according to Claim 9, wherein said primers are DB308 (SEQ ID NO:1), DB309 (SEQ ID NO: 2), DB337 (SEQ ID NO: 4) and DB311 (SEQ ID NO:3).

11. The method according to any one of Claims 1-10, wherein said panel of oligonucleotide probes is fixed to a solid support, each probe being located in a discrete and distinct location on said solid support.

12. A kit for performing the method according to any one of Claims 1-11, which kit comprises said panel of oligonucleotide probes.

13. A kit according to Claim 12 that further comprises a pair of oligonucleotide primers for amplifying said second and/or third exon sequences.

14. A kit according to Claim 12 or 13, wherein said panel of probes comprises two or more probes selected from the group consisting of DB312 (SEQ ID NO: 5), DB313 (SEQ ID NO: 6), DB314 (SEQ ID NO: 7), DB315 (SEQ ID NO: 8), DB316 (SEQ ID NO: 9), DB317 (SEQ ID NO: 10), DB318 (SEQ ID NO: 11), DB319 (SEQ ID NO: 12), DB320 (SEQ ID NO: 13), DB321 (SEQ ID NO: 14), DB322 (SEQ ID NO: 15), DB323 (SEQ ID NO: 16), DB324 (SEQ ID NO: 17), DB325 (SEQ ID NO: 18), DB326 (SEQ ID NO: 19), DB327 (SEQ ID NO: 20), DB328 (SEQ ID NO: 21), DB329 (SEQ ID NO: 22), DB330 (SEQ ID NO: 23), DB331 (SEQ ID NO: 24), DB332 (SEQ ID NO: 25), DB333 (SEQ ID NO: 26), DB334 (SEQ ID NO: 27), DB335 (SEQ ID NO: 28), DB336 (SEQ ID NO: 29), RAP10 (SEQ ID NO: 30), RAP11 (SEQ ID NO: 31), RAP12 (SEQ ID NO: 32), RAP13 (SEQ ID NO: 33), RAP14 (SEQ ID NO: 34), RAP15 (SEQ ID NO: 35), RAP16 (SEQ ID NO: 36), RAP17 (SEQ ID NO: 37), RAP18 (SEQ ID NO: 38), and RAP19 (SEQ ID NO: 39).

15. A kit according to Claim 13 or 14, wherein said primers are DB308 (SEQ ID NO: 1) and DB309 (SEQ ID NO: 2).

16. A kit according to Claim 13 or 14, wherein said primers are DB337 (SEQ ID NO: 4) and DB311 (SEQ ID NO: 3).

17. A kit according to Claim 13 or 14, wherein said primers are DB308 (SEQ ID NO: 1), DB309 (SEQ ID NO: 2), DB337 (SEQ ID NO: 4), and DB311 (SEQ ID NO: 3).
